(19) Europäisches Patentamt
European Patent Office
Office européen des brevets

(11) Veröffentlichungsnummer: **0 143 397**
B1

(12) # EUROPÄISCHE PATENTSCHRIFT

(45) Veröffentlichungstag der Patentschrift: **08.04.87**

(51) Int. Cl.⁴: **C 07 D 307/68,** C 09 K 15/32, C 08 K 5/00

(21) Anmeldenummer: **84113766.4**

(22) Anmeldetag: **14.11.84**

(54) **Furan-3-carbonsäurehydroxamate und deren Verwendung.**

(30) Priorität: **23.11.83 DE 3342219**

(43) Veröffentlichungstag der Anmeldung: **05.06.85 Patentblatt 85/23**

(45) Bekanntmachung des Hinweises auf die Patenterteilung: **08.04.87 Patentblatt 87/15**

(84) Benannte Vertragsstaaten: **CH DE FR GB IT LI NL**

(56) Entgegenhaltungen:
**CHEMICAL ABSTRACTS, Band 81, Nr. 26, Dezember 30, 1974, Seite 549, Zusammenfassung 177685a COLUMBUS, OHIO, (US). J. SHUKLA et al.: "Copper (II), zinc (II), nickel (II), cobalt (II) and manganese (II) chelates of N-phenyl-2-furohydroxamic acid".**
**CHEMICAL ABSTRACTS, Band 99, Nr. 16, Oktober 17, 1983, Seite 39, Zusammenfassung 123512v, COLUMBUS, OHIO, (US) M. RAO et al.: "Photostabilization of poly(methyl methacrylate) by nickel (II) hydroxamates".**

(73) Patentinhaber: **BASF Aktiengesellschaft, Carl-Bosch- Strasse 38, D-6700 Ludwigshafen (DE)**

(72) Erfinder: **Neumann, Peter, Dr., Franz- Schubert-Strasse 1, D-6908 Wiesloch (DE)**
Erfinder: **Thomas, Michael, Dr., Am Wingertsberg 4, D-6719 Weisenheim (DE)**
Erfinder: **Weiss, Stefan, Dr., Carl- Beck- Strasse 46, D-6903 Neckargemuend (DE)**
Erfinder: **Trauth, Hubert, Milanstrasse 5, D-6724 Dudenhofen (DE)**

**EP 0 143 397 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Furan-3-carbonsäurehydroxamate und deren Verwendung zum Stabilisieren von organischen Materialien.

Gegen Licht, Sauerstoff und Temperatur empfindliche organische Materialien, insbesondere Kunststoffe, werden durch eingearbeitete oder als Schutzschicht aufgebrachte Stabilisatoren gegenüber äußeren Angriffen geschützt.

Stabilisatoren für die unterschiedlichsten Anwendungen sind seit langem bekannt.

Aufgabe der vorliegenden Erfindung war es für Thermoplaste, Insbesondere für Polyolefine Stabilisatoren aufzufinden, die eine geringe Eigenfarbe und eine gute Stabilisationswirkung zeigen.

Es wurde gefunden, daß diese aufgabe mit Hilfe der erfindungegemäßen Hydroxamate gelöst wird.

Gegenstand der Erfindung sind Furan-3-carbonsäurehydroxamate der Formel

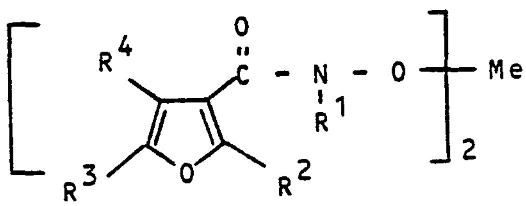

(I),

in der

$R^1$ für $C_1$- bis $C_{18}$-Alkyl, gegebenenfalls durch 1 oder 2 $C_1$- bis $C_4$-Alkyl substituiertes $C_3$- bis $C_8$-Cycloalkyl oder für gegebenenfalls durch $C_1$- bis $C_{12}$-alkyl, Chlor, Brom und/oder $C_1$- bis $C_{12}$-Alkoxy ein- bis dreifach substituiertes Phenyl und wobei die substituenten gleich oder verschieden sein können,

$R^2$ für Methyl oder Ethyl,

$R^3$ und $R^4$ unabhängig voneinander für Wasserstoff, Brom, Chlor, für gegebenenfalls durch Chlor, Brom und/oder $C_1$- bis $C_4$-Alkoxy ein- bis zweifach substituiertes $C_1$- bis $C_{18}$-Alkyl oder für $-CH_2-COOR^5$ stehen, worin $R^5$ ein gegebenenfalls durch Chlor, Brom und/oder $C_1$- bis $C_4$-Alkoxy ein- bis zweifach substituiertes $C_1$- bis $C_{18}$-Alkyl ist, und Me für zweiwertiges Nickel oder Cobalt stehen.

Die Hydroxamate I stabilisieren Kunststoffe, z.B. Thermoplaste, insbesondere Polyolefine wie Polyethylen, Polypropylen und andere Olefin-Homo- und Copolymere, Styrol-homo- und Styrol-Mischpolymerisate, synthetische Kautschuke, Polyvinylbutyral, Ethylen-Vinylacetat-Copolymere, Polyphenylenoxid und deren Gemische mit anderen Kunststoffen gegenüber der Einwirkung von Licht, Sauerstoff und Temperatur. Ein weiterer Vorteil ist, daß wegen der geringen Eigenfarbe von (I) die Polymeren auch bei höheren Anwendungskonzentrationen von (I) praktisch im Farbton nicht verändert werden.

Für $R^1$, $R^3$, $R^4$ und/oder $R^5$ kommen als $C_1$- bis $C_{18}$-Alkyl im einzelnen z.B. in Betracht: Methyl, Ethyl, n-Propyl, iso-Propyl, n-Butyl, iso-Butyl, sek.-Butyl, tert.-Butyl, n-Pentyl, 3-Methylbutyl, 1-Ethylpropyl, 1,1-Di-methylpropyl, 2,2-Dimethylpropyl, n-Hexyl, n-Heptyl, 1-Ethylphenyl, n-Octyl, 2-Ethylhexyl, 2,4,4-Trimethylpentyl, n-Nonyl, n-Decyl, n-Undecyl, n-Tridecyl, n-Pentadecyl, n-Heptadecyl und Octadecyl.

Im Falle von $R^3$, $R^4$ und/oder $R^5$ kommen als Substituenten am Alkyl z.B. In Betracht: Chlor, Brom, Methoxy, Ethoxy, 2-Bromethoxy, 2,2,2-Trichlorethoxy und Isobutoxy.

Als gegebenenfalls durch Alkyl substituiertes Cycloalkyl sind für $R^1$ im einzelnen z.B. zu nennen: Cyclopropyl, Cyclobutyl, Cyclopentyl, Cyclohexyl, 4-Methylcyclohexyl, 4-tert.-Butylcyclohexyl, 3,5-Dimethylcyclohexyl, Cycloheptyl und Cyclooctyl.

Als gegebenenfalls substiuiertes Phenyl kommt für $R^1$ z.B. in Betracht: Phenyl, 2-, 3- und 4-Methylphenyl, 2-, 3- und 4-Ethylphenyl, 2-, 3- und 4-n-Propylphenyl, 2-, 3- und 4-Isopropylphenyl, 2-, 3- und 4-n-Butyl-phenyl, 2-, 3- und 4-(2'-Butyl)phenyl, 2-, 3- und 4-tert.Butylphenyl, 2,3-, 2,4-, 2,5-, 2,6-, 3,4- und 3,5-Dimethylphenyl, 2,4,6-Trimethylphenyl, 2,4-Dimethyl-6-tert.butylphenyl, n-Hexylphenyl, Heptylphenyl, Octylphenyl, (2'-Ethylhexyl)-phenyl, Nonylphenyl, Decylphenyl, Dodecylphenyl, 2-, 3- und 4-Chlorphenyl, 2-, 3- und 4-Bromphenyl, 2,4-, 2,5-, 2,6- und 3,5-Dichlorphenyl, 2-, 3- und 4-Methoxyphenyl, 2-, 3- und 4-Ethoxyphenyl, Propoxyphenyl, n- und i-Butoxyphenyl, Hexoxyphenyl, Octoxyphenyl, Decoxyphenyl, Nonoxyphenyl und Dodecyloxyphenyl.

Bevorzugt sind Hydroxamate der Formel I, in der $R^1$ für Methyl, Ethyl, Cyclohexyl, Phenyl, Chlorphenyl oder Methylphenyl, $R^2$ und $R^3$ für Methyl oder Ethyl, wobei $R^2$ und $R^3$ gleich oder verschieden sein können, $R^4$ für Wasserstoff oder Methyl und Me für zweiwertiges Nickel stehen.

Von diesen sind die Verbindungen I mit $R^1$ = Phenyl, 4-Chlorphenyl, 3-Methylphenyl oder Cyclohexyl, $R^2$ und $R^3$ = Methyl, $R^4$ = Wasserstoff oder Methyl und Me = Ni hervorzuheben. Ganz besonders bevorzugt ist (I) mit $R^1$ = Phenyl oder Cyclohexyl, $R^2$ = $R^3$ = Methyl, $R^4$ = Wasserstoff und Me = Ni.

Außerdem ist aus anwendungstechnischen Gründen die Verbindung (I) mit $R^1$ = Phenyl, $R^2$ = $R^3$ = Methyl, $R^4$ = Wasserstoff und Me = Cobalt bevorzugt.

Die Herstellung der den Hydroxamaten I zugrundeliegenden Hydroxamsäuren ist bekannt (DE-OS 24 55 082). Die Umsetzung zu den Metallsalzen erfolgt nach an sich bekannten Verfahren durch Umsetzen der Hydroxamsäuren mit Me-Salzen. Vorzugsweise erfolgt die Umsetzung in polaren, mit Wasser mischbaren organischen Flüssigkeiten, insbesondere in Methanol, Ethanol oder Isopropanol, von denen Ethanol bevorzugt

ist.

Je nach dem angewendeten Lösungsmittel und/oder den Herstellbedingungen kann das Hydroxamat I 1 oder 2 Mol Kristallwasser enthalten.

Für die Anwendung als Stabilisatoren werden die Hydroxamate I in die zu stabilisierenden Substanzen eingearbeitet oder als Schutzschicht aufgebracht.

Die Konzentration an (I) beträgt 0,05 bis 2, vorzugsweise 0,1 bis 1,0 Gew.%, bezogen auf das zu stabilisierende Material. Als zu stabilisierendes Material kommen vor allem Polyolefine, insbesondere Polyethylen und Polypropylen in Betracht.

Die Einarbeitung von (I) in das Material bzw. die Beschichtung dieser Materialien mit (I) ist dem Fachmann bekannt und erfolgt nach an sich bekannten Verfahren. Die Einarbeitung kann auch zusammen mit anderen Stabilisatoren und gegebenenfalls in solchen Materialien üblichen weiteren Mitteln (Hilfsmitteln) erfolgen.

Die Erfindung soll durch die folgenden Beispiele zusätzlich erläutert werden. Die im folgenden angegebenen Teile sind Gewichtsteile.

A. Herstellung der Hydroxamate

**Beispiel 1**

27,54 Teile des Kaliumsalzes der N-Cyclohexyl-2,5-dimethylfuran-3-hydrox-amsäure werden in 50 Teilen Ethanol bei einer Temperatur von 40 bis 50°C innerhalb von 15 Minuten mit einer warmen Lösung von 26,15 Teilen Nickel-II-chlorid-hexahydrat in 50 Teilen Ethanol versetzt. Die Mischung wird dann 4 Stunden bei einer Temperatur von 40 bis 50°C gerürt. Anschließend filtriert man das ausgefallene Kaliumchlorid heiß ab und engt das Filtrat unter vermindertem Druck auf das halbe Volumen ein. Beim Abkühlen des so aufkonzentrierten Filtrats scheiden sich Kristalle ab, die abgesaugt und getrocknet werden. Man erhält 24,8 Teile des Nickelsalzes der Formel

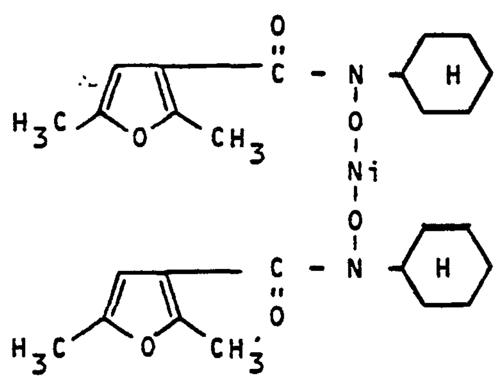

(II)

mit einem Schmelzpunkt von 120 bis 128°C.

**Beispiele 2 bis 4**

Man verfährt wie in Beispiel 1, verwendet jedoch die in der Tabelle angegebenen Hydroxamsäuren in Form der Kaliumsalze. Nach dem Aufarbeiten erhält man die in Spalte 4 angegebene Menge des Nickelsalzes der Hydroxamsäure III mit dem in Spalte 5 angegebenen Schmelzpunkt.

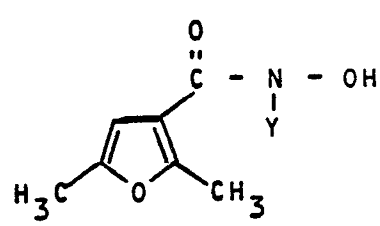

(III)

| Bsp. | Hydroxamsäure (III) Kaliumsalz [Teile] | Y | Nickelhydroxamat Ausbeute [Teile] | Schmp. [°C] |
|---|---|---|---|---|
| 2 | 26,94 | 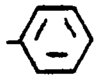 | 24,1 | 270 |
| 3 | 30,39 | 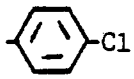 | 27,3 | 164/168 |
| 4 | 28,34 | 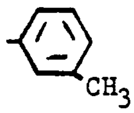 | 25,5 | 220/224 |

**Beispiel 5**

27,54 Teile N-Cyclohexyl-2,5-dimethyl-furan-3-hydroxyamsäure/Kaliumsalz wurden analog Beispiel 1 mit einer warmen Lösung von 26,17 Teilen Kobalt-II-chlorid-hexahydrat in 50 Teilen Ethanol versetzt und aufgearbeitet. Ausbeute: 24,3 Teile des Kobalthydroxamats der Formel

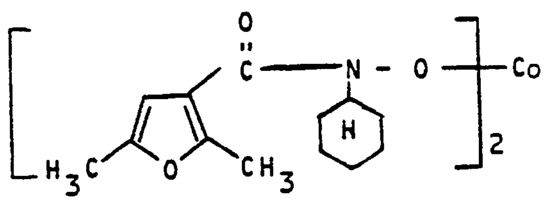

(IV)

Schmp. 59/67° C.

B. Anwendungsbeispiele

**Anwendungsbeispiel 1**

a) 0,25 Teile der Verbindung aus Beispiel 1 werden in 100 Teilen Polyethylen-Pulver durch zweimaliges Extrudieren bei 180°C eingearbeitet und zu 200 µm dicken Platten gepreßt. Nach 14 tägiger Lagerung im Dunkeln bei 25° C zeigt die Oberfläche der Platten keinen Belag.

b) Die nach a) hergestellten Platten werden in einem Q.U.V.-Gerät auf ihre Bewitterungsstabilität getestet. Die Alterung wird durch Messen der "CO-Zahl" nach bestimmten Zeitintervallen bestimmt. Die Meßergebnisse sind in der untenstehenden Tabelle zusammengefaßt.

c) Vergleich mit dem Stand der Technik

Wie in b) werden Polyethylen-Preßplatten mit einem Gehalt von 0,25 % n-Butylamin-Nickel-[2,2'-thiobis(4-tert.-octylphenolat)] bewittert. Die Ergebnisse sind in der folgenden Tabelle zusammengefaßt.

| "CO-Zahl" / Anwendungsbsp. | Bewitterungszeit [h] 0 | 500 | 1000 | 1500 | 2000 |
|---|---|---|---|---|---|
| 1b | 0,2 | 0,25 | 1,3 | 5,4 | 7,55 |
| 1c | 0,2 | 0,25 | 0,4 | 1,3 | 8,25 |

**Anwendungsbeispiel 2**

0,25 Teile der Verbindung aus Beispiel 2 werden in 100 Teilen Polypropylen-Pulver durch zweimaliges Extrudieren bei 180° C eingearbeitet und zu 200 µm dicken Platten gepreßt. Nach 14tägiger Lagerung im Dunkeln bei 25° C zeigt die Oberfläche der Platten keinen Belag.

**Patentansprüche**

1. Furan-3-carbonsäurehydroxamate der Formel

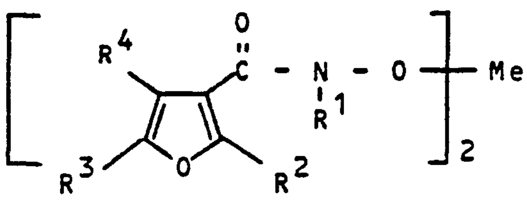

in der

$R^1$ für $C_1$- bis $C_{18}$-Alkyl, gegebenenfalls durch 1 oder 2 $C_1$- bis $C_4$-Alkyl substituiertes $C_3$- bis $C_8$-Cycloalkyl oder für gegebenenfalls durch $C_1$- bis $C_{12}$-Alkyl, Chlor, Brom und/oder $C_1$- bis $C_{12}$-Alkoxy ein- bis dreifach substituiertes Phenyl und wobei die Substituenten gleich oder verschieden sein können,

$R^2$ für Methyl oder Ethyl,

$R^3$ und $R^4$ unabhängig voneinander für Wasserstoff, Brom, Chlor, für gegebenenfalls durch Chlor, Brom und/oder $C_1$- bis $C_4$-Alkoxy ein-bis zweifach substituiertes $C_1$- bis $C_{18}$-Alkyl oder für $-CH_2-COOR^5$ stehen, worin $R^5$ ein gegebenenfalls durch Chlor, Brom und/oder $C_1$- bis $C_4$-Alkoxy ein- bis zweifach substituiertes $C_1$-bis $C_{18}$-Alkyl ist, und

Me für zweiwertiges Nickel oder Cobalt stehen.

2. Furan-3-carbonsäurehydroxamate gemäß Anspruch 1, dadurch gekennzeichnet, daß $R^1$ für Methyl, Ethyl, Cyclohexyl, Phenyl, Chlorphenyl oder Methylphenyl, $R^2$ und $R^3$ unabhängig voneinander für Methyl oder Ethyl, $R^4$ für Wasserstoff oder Methyl und Me für zweiwertiges Nickel stehen.

3. Furan-3-carbonsäurehydroxamate gem. Anspruch 2, dadurch gekennzeichnet, daß $R^1$ für Phenyl, 4-Chlorphenyl, 3-Methylphenyl oder Cyclohexyl und $R^2$ und $R^3$ für Methyl stehen.

4. Furan-3-carbonsäurehydroxamate gem. Anspruch 2 oder 3, dadurch gekennzeichnet, daß $R^4$ Wasserstoff ist.

5. Furan-3-carbonsäurehydroxamate gem. Anspruch 1, dadurch gekennzeichnet, daß $R^1$ für Phenyl oder Cyclohexyl, $R^2$ und $R^3$ für Methyl, $R^4$ für Wasserstoff und Me für Ni stehen.

6. Furan-3-carbonsäurehydroxamat gemäß Anspruch 1, gekennzeichnet durch die Formel

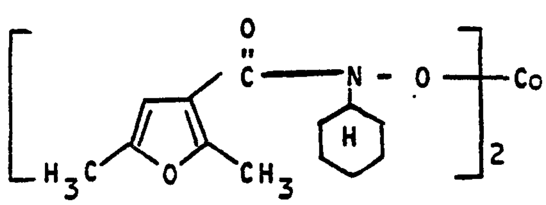

(IV)

7. Verwendung der Furan-3-carbonsäurehydroxamate gemäß den Ansprüchen 1 bis 6 als Stabilisatoren in Kunststoffen, gegebenenfalls in Gegenwart von weiteren, in Kunststoffen üblichen Zusatzstoffen.

## Claims

1. A furan-3-carboxhydroxamate of the formula

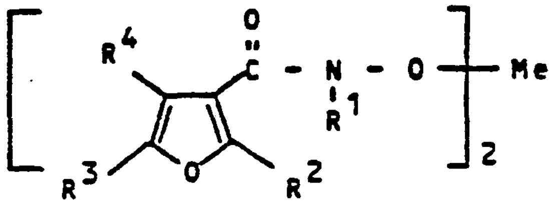

,

where

R is $C_1$-$C_{18}$-alkyl, $C_3$-$C_8$-cycloalkyl which is unsubstituted or monosubstituted or disubstituted by $C_1$-$C_4$-alkyl, or phenyl which is unsubstituted or monosubstituted, disubstituted or trisubstituted by $C_1$-$C_{12}$-alkyl, chlorine, bromine and/or $C_1$-$C_{12}$-alkoxy, and the substituents may be identical or different,

$R^2$ is methyl or ethyl,

$R^3$ and $R^4$ independently of one another are hydrogen, bromine, chlorine, or $C_1$-$C_{18}$-alkyl which is unsubstituted or monosubstituted or disubstituted by chlorine, bromine and/or $C_1$-$C_4$-alkoxy, or are each -$CH_2$-$COOR^5$, where $R^5$ is $C_1$-$C_{18}$-alkyl which is unsubstituted or monosubstituted or disubstituted by chlorine, bromine and/or $C_1$-$C_4$-alkoxy, and

Me is divalent nickel or cobalt.

2. A furan-3-carbohydroxamate as claimed in claim 1, wherein $R^1$ is methyl, ethyl, cyclohexyl, phenyl, chlorophenyl or methylphenyl, $R^2$ and $R^3$ independently of one another are methyl or ethyl, $R^4$ is hydrogen or methyl, and Me is divalent nickel.

3. A furan-3-carbohydroxamate as claimed in claim 2, wherein $R^1$ is phenyl, 4-chlorophenyl, 3-methylphenyl or cyclohexyl and $R^2$ and $R^3$ are each methyl.

4. A furan-3-carbohydroxamate as claimed in claim 2 or 3, wherein $R^4$ is hydrogen.

5. A furan-3-carbohydroxamate as claimed in claim 1, wherein $R^1$ is phenyl or cyclohexyl, $R^2$ and $R^3$ are each methyl, $R^4$ is hydrogen, and Me is Ni.

6. A furan-3-carbohydroxamate as claimed in claim 1, of the formula

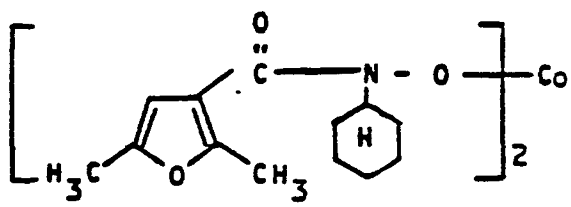

7. The use of a furan-3-carbohydroxamate as claimed in claims 1 to 6 as stabilizer in plastics in the presence or absence of other additives usually used in plastics.

## Revendications

1. Hydroxamates d'acide furanne-3-carboxylique de formule

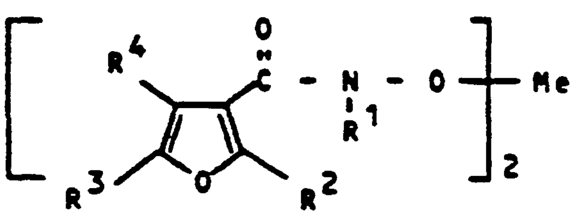

dans laquelle

$R^1$ est mis pour un radical alkyle en $C_1$ à $C_{18}$, un radical cycloalkyle en $C_3$ à $C_8$ éventuellement substitué par 1 ou 2 radicaux alkyle en $C_1$ à $C_4$ ou un radical phényle éventuellement substitué une à trois fois par des radicaux alkyle en $C_1$ à $C_{12}$, des atomes de chlore, de brome et/ou des radicaux alcoxy en $C_1$ à $C_{12}$, les substituants pouvant être identiques ou différents, $R^2$ est mis pour un radical méthyle eu éthyle,

$R^3$ et $R^4$, indépendamment l'un de l'autre, sont mis chacun pour un atome d'hydrogène, de brome, de chlore, pour un radical alkyle en $C_1$ à $C_{18}$ éventuellement substitué une ou deux fois par des atomes de chlore, de brome et/ou des radicaux alcoxy en $C_1$ à $C_4$ ou pour -$CH_2$-$COOR^5$, $R^5$ étant un radical alkyle en $C_1$ à $C_{18}$

éventuellement substitué une ou deux fois par des atomes de chlore, de brome et/ou des radicaux alcoxy en $C_1$ à $C_4$, et Me est mis pour un atome de nickel ou de cobalt bivalent.

2. Hydroxamates d'acide furanne-3-carboxylique selon la revendication 1, caractérisés en ce que $R^1$ est mis pour un radical méthyle, éthyle, cyclohexyle, phényle, chlorophényle ou méthylphényle, $R^2$ et $R^3$, indépendamment l'un de l'autre, sont mis chacun pour un radical méthyle ou éthyle, $R^4$ est mis pour un atome d'hydrogène ou un radical méthyle et Me pour un atome de nickel bivalent.

3. Hydroxamates d'acide furanne-3-carboxylique selon la revendication 2, caractérisés en ce que $R^1$ est mis pour un radical phényle, 4-chlorophényle, 3-méthylphényle ou cyclohexyle et $R^2$ et $R^3$ sont mis chacun pour un radical méthyle.

4. Hydroxamates d'acide furanne-3-carboxylique selon la revendication 2 ou 3, caractérisés en ce que $R^4$ est un atome d'hydrogène.

5. Hydroxamates d'acide furanne-3-carboxylique selon la revendication 1, caractérisés en ce que $R^1$ est mis pour un radical phényle ou cyclohexyle, $R^2$ et $R^3$ sont mis chacun pour un radical méthyle, $R^4$ est mis pour un atome d'hydrogène et Me pour un atome de Ni.

6. Hydroxamate d'acide furanne-3-carboxylique selon la revendication 1, caractérisé par la formule

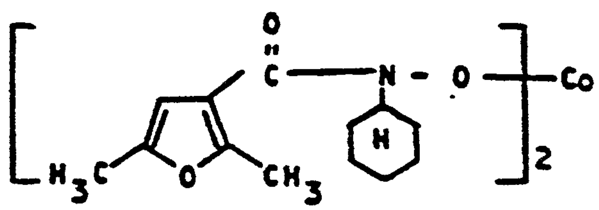

(IV)

7. Utilisation des hydroxamates d'acide furanne-3-carboxylique selon l'une quelconque des revendications 1 à 6 comme stabilisants dans des matières plastiques, le cas échéant en présence d'autres produits auxiliaires utilisés ordinairement dans des matières plastiques.